(19) Europäisches Patentamt
European Patent Office
Office européen des brevets

(11) EP 1 810 656 A1

(12) EUROPEAN PATENT APPLICATION
published in accordance with Art. 158(3) EPC

(43) Date of publication:
25.07.2007 Bulletin 2007/30

(21) Application number: 05795412.5

(22) Date of filing: 13.10.2005

(51) Int Cl.:
*A61K 8/00* (2006.01) *G01N 33/50* (2006.01)
*A61Q 19/00* (2006.01)

(86) International application number:
PCT/JP2005/019266

(87) International publication number:
WO 2006/041214 (20.04.2006 Gazette 2006/16)

(84) Designated Contracting States:
AT BE BG CH CY CZ DE DK EE ES FI FR GB GR HU IE IS IT LI LT LU LV MC NL PL PT RO SE SI SK TR

(30) Priority: 14.10.2004 JP 2004299524
11.10.2005 JP 2005296219

(71) Applicant: Shiseido Company, Limited
Chuo-ku
Tokyo 104-8010 (JP)

(72) Inventors:
- MATSUNAGA, Yukiko, c/o Shiseido Research Center
Yokohama-shi, Kanagawa 236-0004 (JP)
- AMANO, Satoshi, c/o Shiseido Research Center
Yokohama-shi, Kanagawa 236-0004 (JP)
- OGURA, Yuki, c/o Shiseido Research Center
Yokohama-shi, Kanagawa 236-0004 (JP)

(74) Representative: Winkler, Andreas Fritz Ernst
Forrester & Boehmert
Pettenkoferstrasse 20-22
80336 München (DE)

# (54) PREVENTION OR IMPROVEMENT OF WRINKLES BY USING ADAM ACTIVATION INHIBITOR

(57) Awrinklingpreventing or remedying agent contains a substance, which is capable of inhibiting activity of ADAM (a disintegrin and metalloprotease) present in a skin. Wrinkling is prevented or remedied by applying a substance, which is capable of inhibiting activity of ADAM present in a skin, to the skin. Anti-wrinkling effects are evaluated with a method wherein enzymatic activity or a gene expression level of ADAM in a skin, a skin tissue, or cells is used as an index.

EP 1 810 656 A1

Printed by Jouve, 75001 PARIS (FR)

## Description

## Technical Field

This invention relates to wrinkling prevention or remedy with an ADAM activity inhibiting substance. This invention also relates to a method of evaluating anti-wrinkling effects by utilizing ADAM activity inhibition as an index.

## Background Art

In proportion as persons grow old, wrinkles increase as one of skin aging phenomena. Recently, from the view point of beauty culture, or the like, persons, particularly females, take a markedly increasing interest in prevention and remedy of wrinkling. In accordance with wrinkle occurrence sites, wrinkle occurrence mechanisms, and the like, wrinkles may be roughly classified into large wrinkles, fine wrinkles, and crapy wrinkles. The large wrinkles are the deep wrinkles occurring at the backs of the necks, and the like, primarily due to light aging. The fine wrinkles are the comparatively shallow wrinkles occurring about the eyes or the mouths. The crapy wrinkles are the pleat-like wrinkles occurring at unexposed body regions, such as abdomens of old persons.

Heretofore, studies of wrinkling have been conducted with respect to the large wrinkles occurring primarily due to the light aging, and the wrinkling has been taken as one of symptoms due to the light aging. As for the light aging, studies of occurrence mechanisms, and the like, have been advanced, and evaluation systems with animal models or humans have been established. The majority of attempts at suppression of wrinkle formation due to the light aging have heretofore been made as the attempts to prevent or relieve adverse effects of ultraviolet light on the skins. For example, such that the adverse effects of the ultraviolet light on the skins may be prevented, there have been developed and used cosmetic preparations (sun screen cosmetic preparations and sun protect cosmetic preparations) containing various kinds of substances capable of absorbing, scattering, or blocking the ultraviolet light, such as titanium oxide, zinc oxide, a para-methoxy cinnamic acid ester, and a para-amino benzoic acid ester. Also, it has been found that free radicals occurring in the skins due to UV irradiation adversely affect skin-constituting fiber constituents, such as collagen and elastin, and a cosmetic preparation containing an anti-oxidant has been proposed. (Reference may be made to, for example, PCT Japanese Publication No. 2001-508809.) Further, it has been thought that one of primary factors for the light aging of the skins is the phenomenon such that MMP, which decomposes dermal binding tissues, such as collagen and elastin, is induced by the UV irradiation, and such that the dermal binding tissues are disrupted by MMP. In order for the light aging of the skins to be prevented, there has thus been proposed, for example, a composition for suppressing the light aging of the skins, which composition contains a UVA blocking agent, a UVB blocking agent, and an MMP inhibitor, such as retinoid. (Reference may be made to, for example, PCT Japanese Publication No. 2001-520677.)

Recently, females of middle or advanced age take an increasing interest in beauty culture and have an increasing interest in the fine wrinkles occurring about the eyes or the mouths, which fine wrinkles appear due to lowering of water retention capability of the stratum corneum of the epidermis with age or due to a decrease in sebum caused to occur by lowering of secretion of epidermal lipid with age. It is thought that the fine wrinkles are caused to occur by drying of the skins. It is also thought that the fine wrinkles markedly vary from the large wrinkles, which occur due to the light aging, in fine wrinkle occurrence mechanism, and morphological, histological, or biochemical alteration of the fine wrinkles. For example, it has been confirmed with respect to humans that a water content of a stratum corneum and the degree of the small wrinkles have a correlation with each other. (Reference may be made to, for example, a literature of Imokawa, et al., Fragrance Journal; 1992 (11) 29-42.) Further, it has been reported that, in cases where a barrier function is disrupted successively with an unsaturated fatty acid, epidermal wrinkles occur. (Reference may be made to, for example, a literature of JinMasaki, Perfume Transactions; 2001, Vol. 25, No. 1, 34-38.) However, it has not been made clear sufficiently what biochemical alterations occur on a protein level or a gene level in the finely wrinkled skins, and the prevention and the remedy of the fine wrinkles had to rely upon a procedure with protection of the skins from the drying by use of a moisture retention agent, such as glycerol, sorbitol, or a vegetable liquid extract, or a cosmetic preparation containing collagen, or the like. However, only with the protection of the skins from the drying by use of moisture retention agents, or the like, the fine wrinkles are not capable of being prevented and remedied sufficiently. Therefore, there is a strong demand for a substance, which is capable of preventing and remedying the fine wrinkles more efficiently.

In view of the above circumstances, the objects of the present invention are to clarify biochemical alterations taking part in formation of fine wrinkles in skins, to specify a substance capable of suppressing the biochemical alterations, and to prevent or remedy wrinkling more efficiently by use of the specified substance. Another object of the present invention is to provide a method, with which anti-wrinkling effects of a test substance are capable of being evaluated more efficiently and easily by use of the aforesaid suppression of the biochemical alterations as an index.

**Disclosure of Invention**

The inventors have found that, in cases where tape stripping is performed repeatedly on a hairless mouse, and chronic barrier disruption is thus performed, epidermal and dermal alterations identical with the fine wrinkles occurring in the human skins occur in the skin of the mouse. The inventors thus succeeded in forming a finely wrinkled mouse model. The present invention is based upon the findings that, in the finely wrinkled mouse model described above, the epidermal gene expression of a protein (hereinbelow referred to as ADAM), which belongs to ADAM (a disintegrin and metalloprotease) family having a disintegrin and metalloprotease domain, such as ADAM-9, ADAM-10, or ADAM-17, and the epidermal gene expression of HB-EGF (heparin-binding epidermal growth factor-like growth factor) and amphiregulin, which are released by ADAM from the cell membranes and are activated, have been accelerated. The present invention is also based upon the findings that, in cases where an ADAM inhibitor is applied, hyperplasia of the epidermis and the dermis and the wrinkling are capable of being suppressed.

ADAM, such as ADAM-9, ADAM-10, or ADAM-17, are the enzyme, which is present in surfaces of skin cells, and which acts to release the growth factors, such as the HB-EGF, amphiregulin, TNF-$\alpha$, and TGF-$\alpha$, from the cell membranes in the skins and to activate the growth factors described above. It is considered that one of important mechanisms of the formation of the fine wrinkles is the mechanism, in which ADAM is activated in the skins or undergoes expression acceleration in the skins, in which ADAM thus promotes the release and the activation of the growth factors, such as the HB-EGF, and in which the hyperplasia of the epidermis and the dermis is thereby caused to occur. Therefore, it is considered that, in cases where the activity of ADAM in the skins is suppressed, the acceleration of the activity of the HB-EGF, and the like, is capable of being suppressed, the hyperplasia of the epidermis and the dermis is capable of being suppressed, and the wrinkling, particularly the formation of the fine wrinkles, is capable of being prevented or remedied. Figure 1 is an explanatory schematic view showing a mechanism, in which wrinkling is prevented or remedied by inhibition of activity of ADAM in a skin.

In one aspect of the present invention, the present invention provides a wrinkling preventing or remedying agent, containing a substance, which is capable of inhibiting activity of ADAM present in a skin.

In another aspect of the present invention, the present invention provides a method of preventing or remedying wrinkling, comprising the step of applying a substance, which is capable of inhibiting activity of ADAM present in a skin, to the skin. The method of preventing or remedying wrinkling in accordance with the present invention is capable of being used preferably as a beauty culture method.

In a further aspect of the present invention, the present invention relates to the use of a substance, which is capable of inhibiting activity of ADAM, in prevention or remedy of wrinkling. The substance, which is capable of inhibiting activity of ADAM, is capable of being used in beauty culture treatment for preventing or remedying the wrinkling.

In a still further aspect of the present invention, the present invention relates to the use of a substance, which is capable of inhibiting activity of ADAM, in production of a composition for preventing or remedying wrinkling. No limitation is imposed upon the kind of the composition. However, the composition should preferably be one of preparations for external use for skins, such as cosmetic preparations, pharmaceutical preparations, and quasi-drugs. The composition should more preferably be one of the cosmetic preparations.

Examples of ADAM present in the skins include ADAM-9, ADAM-10, ADAM-12, ADAM-15, ADAM-17, and ADAM-19. Particularly, it has been found that ADAM-9, ADAM-10, and ADAM-17 deeply participate in the release and the activation of the cell growth factors.

The term "inhibiting activity of ADAM" as used herein embraces the inhibition of the enzymatic activity of ADAM and arbitrary actions for lowering the activity of ADAM in the skins, such as the actions for inhibiting the gene expression and protein formation.

In another aspect of the present invention, the present invention provides a method of evaluating anti-wrinkling effects, comprising the steps of:

i) bringing a test substance into contact with a skin, a skin tissue, or cells of a human or an animal,
ii) detecting enzymatic activity or a gene expression level of ADAM in the skin, the skin tissue, or the cells, and
iii) evaluating anti-wrinkling effects of the test substance by use of the enzymatic activity or the gene expression level of ADAM as an index.

ADAM may be, for example, ADAM-9, ADAM-10, or ADAM-17.

By way of example, in cases where skin cells, particularly epidermal keratinocytes, are used, the anti-wrinkling effects of a plurality of test substances are capable of being evaluated quickly and efficiently.

The term "anti-wrinkling effects" as used herein means arbitrary effects of preventing the formation of wrinkles or remedying the wrinkles having been formed.

In cases where the substance capable of inhibiting the activity of ADAM is applied to the skins, the activity of ADAM present in the skins, such as ADAM-9, ADAM-10, or ADAM-17, is capable of being inhibited, and the release

and the activation of the growth factors, such as the HB-EGF, in the epidermis and the dermis, which release and activation are induced by the skin barrier disruption occurring due to various primary factors, such as a decrease in sebum and face washing, are capable of being suppressed. The hyperplasia of the epidermis and the dermis is thus capable of being suppressed. The wrinkling, particularly the formation of the fine wrinkles, is thus capable of being prevented or remediedmarkedly efficiently.

Also, with the method of evaluating anti-wrinkling effects in accordance with the present invention, in which the ADAM activity inhibition is used as the index, the anti-wrinkling substance having large effects is capable of being specified efficiently and easily.

## Brief Description of Drawings

Figure 1 is an explanatory schematic view showing a mechanism, in which wrinkling is prevented or remedied by inhibition of activity of ADAM in the epidermis,
Figure 2A is a diagram showing replica images of a mouse skin having been subjected to tape stripping,
Figure 2B is a diagram showing microscopic photographs of a skin tissue slice of the mouse skin, which skin tissue slice has been stained with HE (hematoxylin-eosin),
Figure 3 is a diagram showing alterations of gene expression of ADAM-9, ADAM-17, HB-EGF, and amphiregulin in a finely wrinkled mouse model,
Figure 4A is a diagram showing replica images of skins having been applied with different medicines in a finely wrinkled mouse model,
Figure 4B is a diagram showing microscopic photographs of skin tissue slices of the skins, which skin tissue slices have been stained with HE (hematoxylin-eosin),
Figure 5 is a graph showing anti-wrinkling effects of ADAM activity inhibiting substances, which effects have been obtained from visual judgment, and
Figure 6 is a graph showing anti-wrinkling effects of the ADAM activity inhibiting substances, which effects are expressed by a wrinkle area (%).

## Best Mode of Carrying Out the Invention

ADAM is the polyfunctional protein having two kinds of characteristics, i.e. adhesion and extracellular protein decomposition, and there are at least 30 kinds of families of ADAM. Nowadays, the number of the kinds of the families of ADAM is increasing. ADAM is expressed in various animals and various tissues. Examples of ADAM present in the skins include ADAM-9, ADAM-10, ADAM-12, ADAM-15, ADAM-17, and ADAM-19. Particularly, it has been found that ADAM-9, ADAM-10, and ADAM-17 deeply participate in the release and the activation of the cell growth factors.

The substance capable of inhibiting the activity of ADAM, which substance is capable of being used in the present invention, may be selected from a wide variety of substances, which are capable of lowering the activity of ADAM in the skins. For example, the substance capable of inhibiting the activity of ADAM may be a substance, which is capable of inhibiting the enzymatic activity of ADAM, or a substance, which is capable of inhibiting the ADAM gene expression or the protein formation. Also, the substance capable of inhibiting the activity of ADAM may be a natural substance, such as an animal-derived substance or a plant-derived substance. Alternatively, the substance capable of inhibiting the activity of ADAM may be a synthetic substance.

Examples of the substance capable of inhibiting the activity of ADAM as described above include TAPI-1 (*N*-(R)-(2-(Hydroxyaminocarbonyl)methyl)-4-Methylpentanoyl-L-Na 1-L-Alanine 2-Aminoethyl amide; Immunex, Seattle, WA) and 4-methoxybenzohydroxamic acid. However, the substance capable of inhibiting the activity of ADAM is not limited to the substance examples described above.

The substance, which is capable of inhibiting the activity of ADAM, should preferably be used in preparations for external use for skins. However, the use of the substance, which is capable of inhibiting the activity of ADAM, is not limited to the use applications described above.

In the preparations for external use for skins, only one kind of the substance capable of inhibiting the activity of ADAM may be contained. Alternatively, at least two kinds of the substances capable of inhibiting the activity of ADAM may be blended in combination in the preparations for j h external use for skins. Also, the proportion of the substance capable of inhibiting the activity of ADAM, in which proportion the substance is blended in the preparations for external use for skins, may vary in accordance with the manner of use, the form of the product, and the like, and is not limited to a particular value. However, for example, the blending proportion of the substance, which is capable of inhibiting the activity of ADAM, with respect to the total quantity of the preparation for external use for skins shouldpreferably fall within the range of 0.001% by mass to 10% by mass, should more preferably fall within the range of 0.005% by mass to 5%

by mass, and should most preferably fall within the range of 0.01% by mass to 1% by mass.

The term "preparations for external use for skins" as used herein embraces cosmetic preparations, pharmaceutical preparations, quasi-drugs, and the like. Also, the preparations for external use for skins may take on a wide variety of preparation forms, such as an aqueous solution type, a solubilization type, an emulsion, type, an oil liquid type, a gel type, a paste type, an ointment type, an aerosol type, a water-oil two-layer type, and a water-oil-powder three-layer type. Further, the preparations for external use for skins may take on a form supported on a sheet-shaped base material.

Also, the preparations for external use for skins may take on various product forms and may be used in various use applications. For example, the preparations for external use for skins may be used as the preparations for external use for faces, bodies, and head skins in the product forms of a cosmetic lotion, a milky lotion, a skin cream, and a pack.

When necessary, besides the substance capable of inhibiting the activity of ADAM, the preparations for external use for skins may also contain other arbitrary constituents, which are ordinarily used in preparations for external use for skins, such as the cosmetic preparations and the pharmaceutical preparations. Also, the preparations for external use for skins may be produced by conventional procedures in accordance with the desired preparation forms. For example, the substance capable of inhibiting the activity of ADAM described above and at least one kind of the constituent selected from the constituents described below may be blended together, and the preparations for external use for skins may thereby be prepared.

Specifically, the preparations for external use for skins may contain at least one kind of an ultraviolet light absorber. Examples of the ultraviolet light absorbers include benzoic acid types of ultraviolet light absorbers, such as para-aminobenzoic acid (hereinbelow abbreviated to PABA), a PABAmonoglyceryl ester, an N, N-dipropoxy PABA ethyl ester, an N, N-diethoxy PABA ethyl ester, an N, N-dimethyl PABA ethyl ester, an N, N-dimethyl PABA butyl ester, and an N,N-dimethyl PABA methyl ester; anthranilic acid types of ultraviolet light absorbers, such as homomenthyl-N-acetyl anthranilate; salicylic acid types of ultraviolet light absorbers, such as amyl salicylate, menthyl salicylate, homomenthyl salicylate, octyl salicylate, phenyl salicylate, benzyl salicylate, and p-isopropanolphenyl salicylate; cinnamic acid types of ultraviolet light absorbers, such as octyl cinnamate, ethyl-4-isopropyl cinnamate, methyl-2,5-di-isopropyl cinnamate, ethyl-2,4-di-isopropyl cinnamate, methyl-2,4-di-isopropyl cinnamate, propyl-p-methoxy cinnamate, isopropyl-p-methoxy cinnamate, iso-amyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-$\alpha$-cyano-$\beta$-phenyl cinnamate, 2-ethylhexyl-$\alpha$-cyano-$\beta$-phenyl cinnamate, glyceryl mono-2-ethylhexanoyl-diparamethoxy cinnamate, and a trimethoxycinnamic, acid methylbis(trimethylsiloxane)silylisopentyl ester; 3-(4'-methylbenzylidene)-d,l-camphor; 3-benzylidene-d,l-camphor; urocanic acid; an urocanic acid ethyl ester; 2-phenyl-5-methylbenzoxazole; 2,2'-hydroxy-5-methylphenyl benzotriazole; 2-(2'-hydroxy-5'-t-octylphenyl) benzotriazole; 2-(2'-hydroxy-5'-methylphenyl) benzotriazole; dibenzalazine; di-anisoylmethane; 4-methoxy-4'-t-butyldibenzoylmethane; 5-(3,3-dimethyl-2-norbornylidene)-3-pentan-2-one; and dimorpholino pyridazinone.

Examples of ultraviolet light scattering agents, which may be used, include particles, such as titanium oxide, fine particle titanium oxide, zinc oxide, fine particle zinc oxide, iron oxide, fine particle iron oxide, cerium oxide.

Ordinarily, the ultraviolet light scattering agents are used in the forms of acicular particles, spindle-shaped particles, spherical particles, and granular particles. Also, the ultraviolet light scattering agents should preferably be the fine particles having a particle diameter of at most 0.1$\mu$m.

It is also preferable to use ultraviolet light scattering agents having been subjected to hydrophobic characteristics imparting processing, such as silicone processing with methyl hydrogen polysiloxane or a silane coupling agent; metallic soap processing; fluorine processing with a perfluoroalkylphosphoric acid diethanolamine salt, perfluoroalkylsilane, or the like; or dextrin fatty acid ester processing.

Examples of liquid fats and oils, which may be used, include an avocado oil, a camellia oil, a turtle oil, a macadamia nut oil, a corn oil, a mink oil, an olive oil, a rapeseed oil, a yolk oil, a sesame oil, a persic oil, a wheat germ oil, a *sasanqua* oil, a castor bean oil, a linseed oil, a safflower oil, a cottonseed oil, a perilla oil, a soybean oil, a peanut oil, a tea seed oil, a kaya oil, a rice bran oil, a Chinese tung oil, a Japanese tung oil, jojoba oil, a germ oil, and triglycerol.

Examples of solid fats and oils, which may be used, include a cacao butter, a coconut oil, a horse tallow, a hardened coconut oil, a palm oil, a beef tallow, a sheep tallow, a hardened beef tallow, a palm kernel oil, a pig tallow, a beef bone tallow, a Japanese wax kernel oil, a hardened oil, a beef leg tallow, a Japanese wax, and a hardened castor bean oil.

Examples of waxes, which may be used, include a bees wax, a candelilla wax, a cotton wax, a carnauba wax, a bayberry wax, an insect wax, a whale wax, a montan wax, a rice bran wax, lanolin, a kapok wax, lanolin acetate, liquid lanolin, a sorgo wax, a lanolin fatty acid isopropyl ester, a lauric acid hexyl ester, a reduced lanolin, a jojoba wax, hard lanolin, a shellac wax, a POE lanolin alcohol ether, POE lanolin alcohol acetate, a POE cholesterol ether, a lanolin fatty acid polyethylene glycol, and a POE hydrogenated lanolin alcohol ether.

Examplesofhydrocarbonoils, which may be used, include liquid paraffin, ozocerite, squalane, pristane, paraffin, ceresine, squalene, vaseline, a micro-crystalline wax, a polyethylene wax, and a Fischer-Tropsch wax.

Examples of higher fatty acids, which may be used, include lauric acid, myristic acid, palmitic acid, stearic acid, behenic acid, oleic acid, undecylenic acid, toluic acid, linoleic acid, linolenic acid, eicosapentaenoic acid (EPA), and docosahexaenoic acid (DHA).

Examples of higher alcohols, which may be used, include straight chain alcohols (such as lauryl alcohol, cetyl alcohol, stearyl alcohol, behenyl alcohol, myristyl alcohol, oleyl alcohol, and cetostearyl alcohol) ; and branched chain alcohols (such as a monostearyl glycerol ether (batyl alcohol), 2-decyl tetradecynol, lanolin alcohol, cholesterol, phytosterol, hexyl dodecanol, and octyl dodecanol).

Examples of synthetic ester oils, which may be used, include isopropyl myristate, cetyl octanoate, octyl dodecyl myristate, isopropyl palmitate, butyl stearate, hexyl laurate, myristyl myristate, decyl oleate, hexyl decyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, a dipentaerythritol fatty acid ester, N-alkyl glycol mono-isostearate, neopentyl glycol dicaprate, di-isostearyl malate, glycerol di-2-heptyl undecanoate, trimethylolpropane tri-2-ethyl hexanoate, trimethylolpropane tri-isostearate, pentaerythritol tetra-2-ethyl hexanoate, glycerol tri-2-ethyl hexanoate, glycerol trioctanoate, glycerol tri-isopalmitate, trimethylolpropane tri-isostearate, cetyl-2-ethyl hexanoate, 2-ethyl hexyl palmitate, glycerol trimyristate, tri-2-heptylundecanoic acid glyceride, a castor bean oil fatty acidmethyl ester, oleyl oleate, acetoglyceride, 2-heptyl undecyl palmitate, di-isobutyl adipate, an N-lauroyl-L-glutamic acid-2-octyl dodecyl ester, di-2-heptyl undecyl adipate, ethyl laurate, di-2-ethyl hexyl sebacate, 2-hexyl decyl myristate, 2-hexyl decyl palmitate, 2-hexyl decyl adipate, 2-ethyl hexyl succinate, tri-ethyl citrate, polyoxyethylene, and a polyoxypropylene random polymer methyl ether.

Examples of silicone oils, which may be used, include chain polysiloxanes (such as dimethyl polysiloxane, methyl phenyl polysiloxane, and diphenyl polysiloxane), cyclic polysiloxanes (such as octamethylcyclotetrasiloxane, decamethyl-cyclopentasiloxane, and dodecamethylcyclohexasiloxane), silicone resins in which three-dimensional network structures have been formed, silicone rubber, and various kinds of modified polysiloxanes (such as amino-modified polysiloxanes, polyether-modified polysiloxanes, alkyl-modified polysiloxanes, and fluorine-modified polysiloxanes).

Further, the preparations for external use for skins may contain, for example, moisture retention agents, such as polyethylene glycol, glycerol, 1,3-butylene glycol, erythritol, sorbitol, xylitol, and maltitol; thickening agents, such as cellulose, hydroxyethyl cellulose, hydroxypropyl cellulose, methylhydroxypropyl cellulose, methyl cellulose, carboxymethyl cellulose, quince seeds, carrageenan, pectin, mannan, curdlan, chondroitin sulfate, starch, galactan, dermatan sulfate, glycogen, gum arabic, heparan sulfate, hyaluronic acid, sodium hyaluronate, tragacanth gum, keratan sulfate, chondroitin, xanthane gum, mucoitin sulfate, hydroxyethyl guar gum, carboxymethyl guar gum, guar gum, dextran, kerato-sulfate, locust bean gum, succinoglucan, charonin acid, chitin, chitosan, carboxymethyl chitin, and agar; lower alcohols, such as ethanol; anti-oxidants, such as butyl hydroxy toluene, tocopherol, and phytin; anti-microbial agents, such as benzoic acid, salicylic acid, sorbic acid, a para-hydroxybenzoic acid alkyl ester, and hexachlorophene; organic acids, such as acyl sarcosine acid (e.g., sodium lauroyl carcosine), glutathione, citric acid, malic acid, tartaric acid, and lactic acid; vitamins, such as vitamin A and derivatives thereof, vitamin B, e.g., vitamin $B_6$ hydrochloride, vitamin $B_6$ tripalmitate, vitamin $B_6$ dioctanoate, vitamin $B_2$ and derivatives thereof, vitamin $B_{12}$, vitamin $B_{15}$ and derivatives thereof, vitamin C, e.g., ascorbic acid, an ascorbic acid sulfuric acid ester (salt), an ascorbic acid phosphoric acid ester (salt), and ascorbic acid dipalmitate, vitamin E, e.g., $\alpha$-tocopherol, $\beta$-tocopherol, $\gamma$-tocopherol, and vitamin E acetate, vitamin D, vitamin H, pantothenic acid, and pantethine; various kinds of medicines, such as nicotinic acid amide, benzyl nicotinate, $\gamma$-oryzanol, allantoin, glycyrrhizinic acid (salt), glycyrrhetic acid and derivatives thereof, hinokitiol, bisabolol, eucalyptol, thymol, inositol, saponins, e.g., saikosaponin, carrot saponin, loofah saponin, and mucrodisaponin, a pantothenyl ethyl ether, ethynyl estradiol, tranexamic acid, arbutin, cepharanthin, and a placenta extract; extracts of vegetables, such as *Rumex japonicus* Houtt., *Sophora flavescens* Aiton, *Nuphar japonicum* DC., orange, *Salvia officinalis* L., *Achillea alpina* L., *Malva sylvestris* L. *var. mauritiana* Mill. (Tree Mallow), Swertie Herb, *Thymus vulgaris* L. (Common Thyme), Japanese Angelica Root, Bitter Orange Peel, birch, *Equisetum arvense* L., loofah, *Aesculus hippocastanum* L (Horse Chestnut), *Saxifraga stolonifera* Meerb., *Arnica montana* L. (Arnica), lily, *Artemisia Princeps* Pampan., *Paeonia lactiflora* Pall., Aloe, *Gardenia jasminoides* Ellis forma *grandiflora* (Lour.) Makino, *Chamaecyparis pisifera* Endl., an extract of *Crataegus oxyacantha* L. (English Hawthorn), an extract of *Hypericum perforatum* L., an iris in extract, a Gambir extract, an extract of *Ginkgo biloba* L. (Ginkgo), an extract of *Thymus quinquecostatus* Celak., an extract of *Foeniculum vulgare* Mill. (Fennel), an oolong tea extract, a water lily extract, a Rose Fruit extract, an extract of *Isodon japonicus* (Burm.) Hara, a Scutellaria Root extract, a Phellodendron Bark extract, an extract of *Lamium album* L. var. *barbatum* (Sieb. et Zucc.) Franch. et Savat., a Glycyrrhiza Extract, an extract of *Gardenia jasminoides* Ellis forma *grandiflora* (Lour.) Makino, a black tea extract, a *Chinese Tamarisk* Twing extract, an extract of *Potentilla tormentilla* Schrank, a rose extract, a loofan extract, a peppermint extract, a rosemary extract, and a royal jelly extract; coloring matter; nonionic surface active agents, such as sorbitanmonolaurate, sorbitan monopalmitate,sorbitansesquioleate,sorbitan trioleate, polyoxyethylene sorbitan monolaurate, polyoxyethylene sorbitan monostearate, polyethylene glycol monooleate, a polyoxyethylene alkyl ether, a polyglycol diether, lauroyl diethanol amide, fatty acid isopropanol amide, a maltitol hydroxy fatty acid ether, alkylated polysaccharides, alkyl glycosides, and a sugar ester; cationic surface active agents, such as stearyl trimethyl ammonium

chloride, benzalkonium chloride, and lauryl amine oxide; anionic surface active agents, such as sodium palmitate, sodium laurate, sodium laurylate, potassium lauryl sulfate, an alkylsulfuric acid triethanol amine ether, a Turkey red oil, linear dodecylbenzene sulfate, polyoxyethylene hardened castor bean oil maleate, and acyl methyl taurine; amphoteric surface active agents; neutralizing agents; anti-oxidants, such as γ-tocopherol, and butyl hydroxy toluene; and antiseptics, such as phenoxy ethanol and paraben (para-hydroxybenzoate).

In the method in accordance with the present invention, the aforesaid substance capable of inhibiting the activity of ADAM may be applied to the skins in one of various forms, with which the substance is capable of being applied to the skins, and with which the purposes of the present invention are capable of being achieved. Also, the aforesaid substance capable of inhibiting the activity of ADAM maybe applied alone. Alternatively, the aforesaid substance capable of inhibiting the activity of ADAM may be applied by being blended with other arbitrary constituents. Further, no limitation is imposed upon the site of the skin, to which site the aforesaid substance capable of inhibiting the activity of ADAM is applied. Specifically, the aforesaid substance capable of inhibiting the activity of ADAM may be applied to every site of the skin on the body surface, including the head skin. The method in accordance with the present invention should preferably be used as the beauty culture method, but is not limited to the use as the beauty culture method.

The method of evaluating anti-wrinkling effects in accordance with the present invention comprises the step of bringing the test substance into contact with the skin, the skin tissue, or the skin cells of the human or the animal.

The method of evaluating anti-wrinkling effects in accordance with the present invention may be carried out by use of one of various skins of the humans and the animals, with which the purposes of the present invention are capable of being achieved. For example, the method of evaluating anti-wrinkling effects in accordance with the present invention may be carried out by use of the skin of a hairless mouse, which skin has been subjected to tape stripping for peeling off the stratum corneum of the skin. Specifically, the skin of the hairless mouse may be subjected to the tape stripping, the stratum corneum of the skin may thus be peeled off, and the skin barrier may thereby be disrupted successively. In such cases, the fine wrinkles occur with the skin of the mouse, and the enzymatic activity or the gene expression of ADAM, such as ADAM-9, ADAM-10, or ADAM-17, is accelerated. It is considered that one of the causes for the formation of the fine wrinkles is the mechanism, in which the release and the activation of the growth factors, such as the HB-EGF, are promoted by the acceleration of the activity of ADAM, and in which the hyperplasia of the epidermis and the dermis is thereby caused to occur. Therefore, it is considered that, with the method of evaluating anti-wrinkling effects in accordance with the present invention, in cases where, for example, a substance, which is capable of lowering the gene expression of ADAM having been accelerated by the chronic barrier disruption described above, is specified, the anti-wrinkling substance is capable of being specified efficiently.

The skin cells used in the method of evaluating anti-wrinkling effects in accordance with the present invention are the epithelial cells, which forms the epidermal areas of the skins. Examples of the epithelial cells include epidermal cells, such as epidermal keratinocytes, sebaceous gland cells, mammary gland cells, epithelial cells of small intestine, and other mucosa epithelial cells. Of the examples of the epithelial cells enumerated above, the epidermal keratinocytes, which are one example of the epidermal cells, have the advantages in that the cultured cells are easily available. Also, this detection method is the technique related to the state of the epidermis. Therefore, the epidermal keratinocytes are the skin cells preferable for this method. Also, the skin cell furnishing source should preferably be the human. Further, the skin cells used in this method should preferably be the cells having been cultured. The cells having been cultured are capable of being prepared by conventional procedures.

For example, in cases where the skin cells are the human epidermal keratinocytes, the human epidermis may be prepared from a surplus skin slice, which is obtained from restorative surgery, dermatology, surgical operations, or the like, with processing, wherein fat tissues and blood are removed from the skin slice, and wherein a dermal section is separated with protease treatment, or the like. Also, the epidermal keratinocytes may be separated from the thus prepared human epidermis and may then be subj ected to primary culture performed by the conventional procedures utilizing a KGM culture medium, or the like. Thereafter, the primary culture cells of the human epidermal keratinocytes may be subj ected to subculture by use of protease, or the like, in accordance with the conventional procedures. In this manner, the desired cultured human epidermal keratinocytes are capable of being obtained. The epidermal keratinocytes of the type described are commercially available. In the present invention, the commercially available epidermal keratinocytes may also be used.

The detection of the enzymatic activity of ADAM is capable of being performed by, for example, measuring the release quantity of the ADAM substrate, such as HB-EGF.

Also, in order for the detection of the gene expression level to be detected, for example, cDNA may be amplified and/or measured by use of RT-PCR, a Northern blotting technique, or the like. Alternatively, the protein may be detected and/or measured by use of ELISA, a Western blotting technique, or the like.

The step of evaluating the anti-wrinkling effects of the test substance by use of the enzymatic activity or the gene expression level of ADAM as the index may comprise, for example, a technique, wherein the skin, the skin tissue, or the skin cells, which have not been added with the test substance, or which have been added with a substance that is known not to affect the activity of ADAM, are utilized as the control, and wherein the enzymatic activity or the gene

expression level of ADAM at the time, at which the test substance has been added, is compared with the enzymatic activity or the gene expression level of ADAM in the control. Bywayof example, a test substance, which has been found as lowering the enzymatic activity or the gene expression level of ADAM as a result of the comparison with the enzymatic activity or the gene expression level of ADAM in the control, may be specified as the substance having the anti-wrinkling effects (i.e., the anti-wrinkling substance or the wrinkling preventing or remedying agent).

No limitation is imposed upon the manner of use of the method of evaluating anti-wrinkling effects in accordance with the present invention. For example, the method of evaluating anti-wrinkling effects in accordance with the present invention may be used for screening and evaluation of an anti-wrinkling substance, or the like.

The present invention will further be illustrated by the following non-limitative examples.

Examples

Preparation of finely wrinkled mouse model

It is considered that, in cases where the skin barrier is weakened or disrupted by various primary factors, such as a decrease in sebum and face washing, and water evaporation from the skin surface increases, the skin dries, and the fine wrinkles are formed. Therefore, tape stripping was repeatedly performed on the skin of a hairless mouse, the stratum corneum of thus peeled off, and the skin barrier was broken. In this manner, a finely wrinkled mouse model was prepared. Specifically, the tape stripping with cellophane adhesive tapes was performed on the skin on the left side of the dorsal section of a hairless mouse (HR-1, male, six weeks of age, supplied by Hoshino Experimental Animals) with the number of times of the tape stripping being adjusted such that a transepidermal water loss (TEWL) (as measured by use of a water evaporation quantity measuring apparatus MEECO, supplied by Meeco Co., U.S.A) might fall within the range of 4mg/cm$^2$/h to 6 mg/cm$^2$/h. Ordinarily, at the initial stage, the tape stripping was iterated approximately four times. At the final stage, the tape stripping was iterated seven to eight times. The tape stripping was performed in three stages per week and was continued for four weeks. The right side of the dorsal section of the hairless mouse was left to stand as a non-treatment area.

Figure 2A is a diagram showing replica images of the mouse skin having been subjected to the tape stripping in the manner described above. Figure 2B is a diagram showing microscopic photographs of a skin tissue slice of the mouse skin, which skin tissue slice has been stained with HE (hematoxylin-eosin)., As illustrated in the replica images (Figure 2A), skin ditches became deep at the stage 24 hours (24h) after the beginning of the tape stripping and at the stage 48 hours (48h) after the beginning of the tape stripping. At the stage one week (1W) after the beginning of the tape stripping, the directions of the skin ditches coincided with predetermined directions. At the stage four weeks (4W) after the beginning of the tape stripping, a wrinkle-like appearance occurred. Also, as illustrated in the microscopic photographs of the skin tissue (Figure 2B), when a comparison was made with the non-treatment area (NT), it was found that the epidermis begun to undergo hyperplasia at the stage 24 hours after the beginning of the tape stripping. At the stage two weeks (2W) after the beginning of the tape stripping, the epidermal hyperplasia was kept. Table 1 shown below lists morphological alterations, histological alterations, and the like, of the finely wrinkled mouse model having been prepared in the manner described above together with the morphological alterations, histological alterations, and the like, of a photo-aged mouse model having been prepared with UV light irradiation (for 10 weeks).

Table 1

| Comparison between finely wrinkled mouse model and photo-aged mouse model | | |
|---|---|---|
| | Finely wrinkled mouse model | Photo-aged mouse model |
| Appearance | Wrinkles were formed in early stages. However, the wrinkles were shallow wrinkles and were remedied soon. | Wrinkles were formed at the stage of about fifth week of UV light irradiation. The wrinkles were deep wrinkles and were not remedied easily. |
| Stratum corneum thickness | Hyperplasia | Hyperplasia |
| Epidermal thickness | The epidermis thickened 2 to 3 times soon, but did not thicken even further. | The epidermis thickened 5 to 6 times. |
| Dermal thickness | Hyperplasia. | Hyperplasia. |
| Inflammatory cytokine | Increased. | Increased. |
| Basement membrane | No alteration occurred. | Several layers to double membrane and rupture occurred. |

(continued)

| Comparison between finely wrinkled mouse model and photo-aged mouse model | | |
|---|---|---|
| | Finely wrinkled mouse model | Photo-aged mouse model |
| Dermal blood vessels | Expanded and increased. | Expanded and increased. |
| Collagen density | No alteration occurred. | Became low. |

The skin of the mouse having been subjected to the tape stripping in the manner described above exhibited the alterations identical with the alterations in the cases of the fine wrinkles of the human skin. Also, the skin of the mouse having been subjected to the tape stripping in the manner described above varied markedly in wrinkle appearance, epidermal hyperplasia, and the like, from the photo-aged mouse model having been prepared with the UV light irradiation. It was thus suggested that, with the method described above, the finely wrinkled mouse model, which markedly varied from the largely wrinkled mouse model having been prepared with the light aging, was capable of being prepared.

Studies of biochemical alterations of finely wrinkled mouse model

With respect to the finely wrinkled mouse model having been prepared in the manner described above, it was presumed that the hyperplasia of the epidermis and the dermis had occurred, and that the cell growth factors had been activated in the epidermis and the dermis due to excitement by the barrier disruption caused to occur by the tape stripping. Therefore, studies were made with respect to the gene expression of HB-EGF and amphiregulin, which were the cell growth factors present in the epidermis, and the gene expression of ADAM-9 and ADAM-17, which were the enzymes acting to release the aforesaid growth factors from the cell membrane and to activate the growth factors.

Measurement of the gene expression quantity was made by use of the RT-PCR method. Specifically, total RNA was extracted from dorsal skin samples, which had been sampled with the passage of time from the left side (tape stripping treatment: T) and the right side (non-treatment: N) of the finely wrinkled mouse model having been prepared in the manner described above. Also, after cDNA had been prepared, RT-PCR was performed by use of a primer specific to the gene of the test object. Further, for reference, the measurement was made in the same manner as that described above with respect to glyceraldehydes-3-phosphate-dehydrogenase (GAPDH) of a housekeeping gene having characteristics such that the expression quantity per cell coincided with a predetermined quantity.

The results as shown in Figure 3 were obtained. As illustrated in Figure 3, as for ADAM-9 and ADAM-17, the gene expression was accelerated at the stage 24 hours after the tape stripping had been performed on the skin and at the stage 4 hours after the tape stripping had been performed on the skin. Also, as for HB-EGF and amphiregulin belonging to the same HB-EGF family, which are released and activated by ADAM, the gene expression was accelerated at the stage 24 hours after the tape stripping had been performed on the skin to the stage one week after the tape stripping had been performed on the skin.

Studies of anti-wrinkling effects of medicine application on finely wrinkled mouse model

Studies were made with respect to the anti-wrinkling effects of the application of various medicines, which were listed in Table 2 below, on the finely wrinkled mouse model.

Table 2

| Medicine | Effects | Supplier |
|---|---|---|
| Control (ethanol: water=1:1) | | |
| 2mM TAPI-1 | ADAM inhibitor | Immunex, Seattle, WA |
| 1% CGS27023A* | MMP inhibitor | Synthesized by the inventor's company |
| 10% Glycerol | Moisture retention agent | Wako Pure Chemical Industries, Ltd., Osaka |
| 1% Oleicacid | Skin roughening promotion | Nakaraitesk, Kyoto |
| *:N-Hydroxy-2-[[(4-methoxyphenyl)sulfonyl]3-picolyl]amino]-3-m ethylbutane amide hydrochloride] (J. Med. Chem. 1997, Vol. 40, pp. 2525-2532) | | |

Specifically, the tape stripping was performed on a hairless mouse in the manner described above. After each tape stripping processing, 100μl of each of the medicines listed in Table 2 was applied to the skin of the hairless mouse.

Figure 4A is a diagram showing replica images of skins having been applied with different medicines in the finely wrinkled mouse model, which replica images have been taken at stages one week, two weeks, and four weeks after the application of each of the medicines. Figure 4B is a diagram showing microscopic photographs of skin tissue slices of the skins at the stage four weeks after the application of each of the medicines, which skin tissue slices have been stained with HE (hematoxylin-eosin). Table 3 below shows the results obtained in accordance with the evaluation criteria described below.

○: Remedied.
Δ: No alteration was found.
×: Aggravated.

Table 3

| Anti-wrinkling effects of various medicines on finely wrinkled mouse model | | | |
|---|---|---|---|
| Medicine | Wrinkling suppressing effects | Epidermal hyperplasia suppressing effects | Dermal hyperplasia suppressing effects |
| Control (ethanol: water=1: 1) | Δ | Δ | Δ |
| 2mM TAPI-1 | ○ | ○ | ○ |
| 1% CGS27023A | Δ | Δ | Δ |
| 10% Glycerol | Δ | × | Δ |
| 1% Oleic acid | × | × | × |

TAPI-1 acting as the ADAM activity inhibiting substance suppressed the wrinkle formation in the cases of the finely wrinkled mouse model and markedly suppressed the hyperplasia of the epidermis and the dermis. Each of CGS27023A (N-hydroxy-2-[[(4-methoxyphenyl) sulfonyl]3-picolyl]amino]-3-methylbutane amide hydrochloride]) (J. Med. Chem. 1997, Vol. 40, pp. 2525-2532) acting as the MMP inhibitor, which exhibited the wrinkle formation preventing effects with respect to the photo-aged mouse model, and glycerol acting as the moisture retention agent was not capable of suppressing the wrinkle formation at all with respect to the finely wrinkled mouse model and was not capable of suppressing the hyperplasia of the epidermis and the dermis. Also, oleic acid, which is the unsaturated fatty acid having the skin roughening promoting action, promoted the wrinkle formation and accelerated the hyperplasia of the epidermis and the dermis.

From the results described above, it was suggested that, only with the moisture retention agent, the formation of the fine wrinkles is not capable of being prevented or remedied sufficiently. It was also suggested that, in cases where the activity of ADAM in the skin is inhibited, and the release and the activation of the growth factors are thereby inhibited, the hyperplasia of the epidermis and the dermis is capable of being suppressed, and the formation of the fine wrinkles is capable of being prevented or remedied efficiently. Further, from the results such that CGS27023A acting as the MMP inhibitor, which exhibited the wrinkle formation suppressing effects with respect to the photo-aged mouse model, was not capable of suppressing the wrinkle formation, it was suggested that the suppression of the wrinkle formation by the substance capable of inhibiting the activity of ADAM in accordance with the present invention is the effects obtained by a mechanism different from the mechanism of the MMP inhibitor.

Searching of novel ADAM activity inhibiting substance and evaluation of anti-wrinkling effects

A novel compound capable of inhibiting the ADAM activity was searched, and the anti-wrinkling effects of the novel compound with respect to the finely wrinkled mouse model were evaluated. Unless otherwise specified, the blending proportion of the compound was expressed in terms of % by mass.

(1) Screening of ADAM activity inhibiting substance

Firstly, the screening of a compound having the ADAM enzyme inhibiting activity was performed by use of HB-EGF-AP/HT-1080 (human fibrosarcoma-derived cultured cells HT-1080 having been modified so as to achieve forcible expression of a fusion protein, in which heat-resistant alkaline phosphatase (AP) has been added to an N terminal of human HB-EGF). On the surfaces of the cells of the used cell strain HB-EGF-AP/HT-1080, the HB-EGF entire length molecule is expressed in the form fused with the alkaline phosphatase. In cases where the cells are excited by a phorbol

ester, the ADAM enzyme on the cell membrane surfaces is activated to cut the HB-EGFmolecule. Since alkaline phosphatase has been bound to HB-EGF having been cut into the released form, in cases where the alkaline phosphatase activity in a culture supernatant is measured, the ADAM enzyme inhibiting activity of the compound is capable of being measured indirectly.

Specifically, HB-EGF-AP/HT-1080, in which the number of cells had been adjusted at $2.0\times10^5$ cells/ml, was sowed at a rate of 0.2ml/well to a 96-well culture micro-plate and was cultured overnight at a temperature of 37˚C. After removal of the culture medium and the washing with PBS (-), a culture medium containing the test substance was added at a rate of 0.1ml/well, and incubation was performed at a temperature of 37˚C for 30 minutes. The pre-treatment was thus performed. Thereafter, the supernatant was removed, and a culture medium, which contained the test substance and 60nM TPA (phorbol ester: 12-o-tetradecanoylphorbol-acetate; Sigma P8139), was added at a rate of 0.2ml/well. The incubation was then performed for 60 minutes, and treatment was thus performed. After the treatment, 0.1ml of the culture supernatant in each well was transferred to awellof amicro-plate for alkaline phosphatase activitymeasurement. Also, the incubation was performed at a temperature of 65˚C for 10 minutes, and endogenous alkaline phosphatase was thus deactivated. Further, a 1mg/ml AP substrate (*p*-nitrophenylphosphate, Wako; 141-02341) was added to each well at a rate of 0.1ml/well, and an absorbance in each well at a wavelength of 405nm was measured immediately. After the incubation was performed at the room temperature for two hours under a light blocking condition, the absorbance in each well at the wavelength of 405nm was measured again. The value obtained by subtracting the absorbance, which was measured at the stage immediately after the addition of the AP substrate, from the absorbance, which was measured at the stage after the two-hour incubation, was taken as the absorbance of each well. An inhibition rate (%) was calculated with the formula shown below.

$$\text{Inhibition rate (\%)} = (A0-AS)/(A0-A100)*100$$

wherein A0 represents the absorbance of the 0% inhibition control (the culture medium containing TPA alone), A100 represents the absorbance of the 100% inhibition control (the culture medium alone), and AS represents the absorbance of the sample.

As a result, high effects of HB-EGF release suppression was found with 4-methoxybenzohydroxamic acid, and it was suggested that 4-methoxybenzohydroxamic acid was capable of inhibiting the ADAM activity. 4-Methoxybenzohydroxamic acid may be represented by the structural formula shown below. Also, the release inhibition rate of 4-methoxybenzohydroxamic acid is listed in Table 4 below.

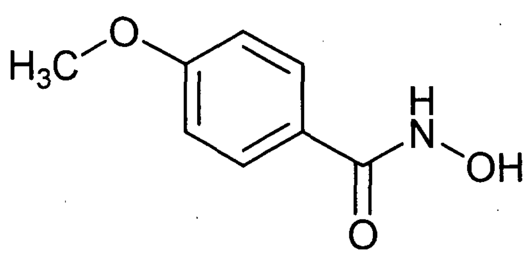

4-Methoxybenzohydroxamic acid

Table 4

| Test substance | Inhibition rate (%) | |
|---|---|---|
| | Concentration | |
| | 10µg/ml | 50µg/ml |
| 4-Methoxybenzohydroxamic acid | 14.8 | 55.8 |

(2) Evaluation of anti-wrinkling effects of ADAM activity inhibiting substance

The anti-wrinkling effects of TAPI-1 and 4-methoxybenzohydroxamic acid acting as the ADAM activity inhibiting substances were studied by use of the finely wrinkled mouse model.

Specifically, on the skin on the left side of the dorsal section of a hairless mouse (HR-l, male, six weeks of age, supplied by Hoshino Experimental Animals), the tape stripping was performed in three stages per week and was continued for four weeks such that the transepidermal water loss (TEWL) (as measured by use of a water evaporation quantity measuring apparatus MEECO, supplied by Meeco Co., U.S.A) might fall within the range of $4mg/cm^2/h$ to $8mg/cm^2/h$. Immediately after each stage of the tape stripping processing had been performed, 100μ1 of TAPI-1 or 4-methoxybenzohydroxamic acid was applied to the skin on the left side of the dorsal section of the mouse. At this time, 4-methoxybenzohydroxamic acid was dissolved in a 50% aqueous ethanol solution such that the concentration of 4-methoxybenzohydroxamic acid might become equal to 1%, and 4-methoxybenzohydroxamic acid was used in the form of the resulting solution. Also, TAPI-1 (supplied by Peptide Research Institute) was evaluated at a concentration of 1mM. Further, as a negative control, a 50% aqueous ethanol solution (i.e., a vehicle) was applied in the same manner as that described above.

The state of occurrence of wrinkles after a period of four weeks had elapsed was expressed by scores with visual judgment. The state of occurrence of wrinkles was evaluated with the rating described below.

0: No wrinkle
1: shallow wrinkles
2: clear wrinkles
3: Deep wrinkles

The state of occurrence of wrinkles was expressed by the scores at intervals of 0.5. A larger score represents deeper wrinkles. The mean value and the standard deviation of each group were calculated. The results illustrated in Figure 5 were obtained.

As illustrated in Figure 5, the mean value of the score of the vehicle was equal to 1.17. Also, the mean value of the score of TAPI-1 was equal to 0.43, and the mean value of the score of 4-methoxybenzohydroxamic acid was equal to 0.91. Each of TAPI-1 and 4-methoxybenzohydroxamic acid acting as the ADAM activity inhibiting substances exhibited significant wrinkling suppression effects in contrast to the vehicle.

Thereafter, the replicas of the dorsal section of the mouse were analyzed by use of a wrinkle analyzing apparatus (supplied by Hamano Engineering), and the wrinkle area (%) was calculated. A low wrinkle area percentage represents that the wrinkle formation has been suppressed. The results illustrated in Figure 6 were obtained.

As clear from Figure 6, the mean value of the wrinkle area (%) of TAPI-1 and the mean value of the wrinkle area (%) of 4-methoxybenzohydroxamic acid were significantly lower than the mean value of the wrinkle area (%) of the vehicle. Each of TAPI-1 and 4-methoxybenzohydroxamic acid acting as the ADAM activity inhibiting substances thus exhibited high wrinkling suppression effects.

From the results described above, it was suggested that each of TAPI-1 and 4-methoxybenzohydroxamic acid acting as the ADAM activity inhibiting substances is capable of suppressing the release of HB-EGF, which acts as the epidermal growth factor, through the inhibition of the ADAM enzyme and is capable of preventing or remedying the wrinkle formation.

## Claims

1. A wrinkling preventing or remedying agent, containing a substance, which is capable of inhibiting activity of ADAM (a disintegrin and metalloprotease) present in a skin.

2. A wrinkling preventing or remedying agent as defined in Claim 1 wherein ADAM is selected from the group consisting of ADAM-9, ADAM-10, and ADAM-17.

3. Use of a substance, which is capable of inhibiting activity of ADAM, in prevention or remedy of wrinkling.

4. Use as defined in Claim 3 wherein the substance, which is capable of inhibiting activity of ADAM, is applied to a skin.

5. Use of a substance, which is capable of inhibiting activity of ADAM, in production of a composition for preventing or remedying wrinkling.

6. Use as defined in Claim 5 wherein the composition is one of cosmetic preparations.

7. Use as defined in Claim 3, 4, 5, or 6 wherein ADAM is selected from the group consisting of ADAM-9, ADAM-10, and ADAM-17.

**8.** A method of evaluating anti-wrinkling effects, comprising the steps of:

i) bringing a test substance into contact with a skin, a skin tissue, or cells of a human or an animal,
ii) detecting enzymatic activity or a gene expression level of ADAM in the skin, the skin tissue, or the cells, and
iii) evaluating anti-wrinkling effects of the test substance by use of the enzymatic activity or the gene expression level of ADAM as an index.

**9.** A method of evaluating anti-wrinkling effects as defined in Claim 8 whereinADAM is selected from the group consisting of ADAM-9, ADAM-10, and ADAM-17.

**10.** A method of evaluating anti-wrinkling effects as defined in Claim 8 or 9 wherein epidermal keratinocytes are used.

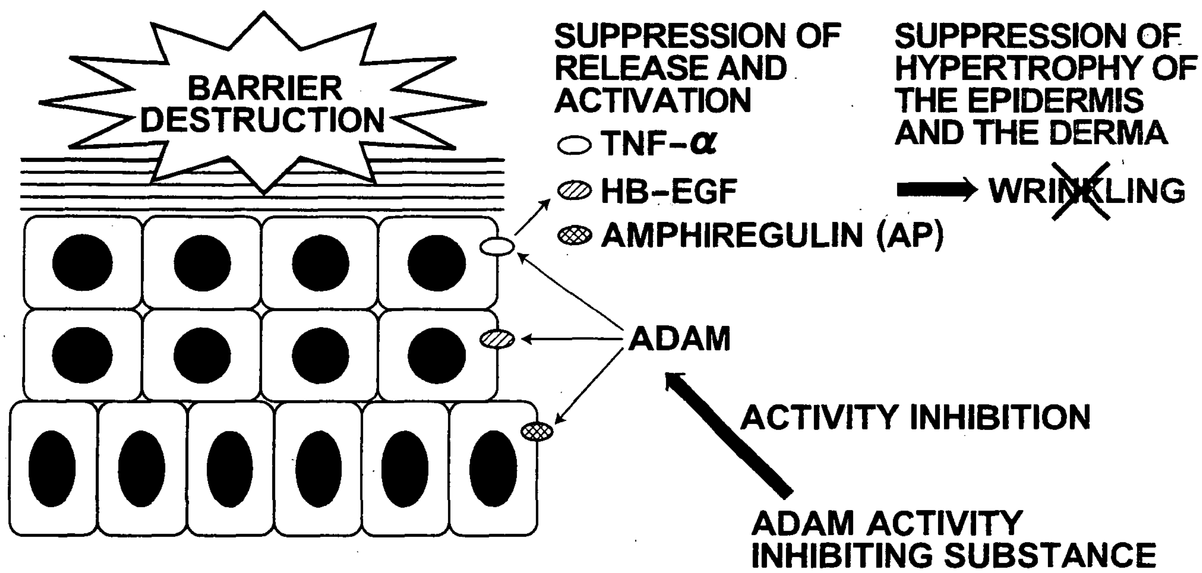
BARRIER
DESTRUCTION
SUPPRESSION OF
RELEASE AND
ACTIVATION
TNF-α
HB-EGF
AMPHIREGULIN (AP)
SUPPRESSION OF
HYPERTROPHY OF
THE EPIDERMIS
AND THE DERMA
WRINKLING
ADAM
ACTIVITY INHIBITION
ADAM ACTIVITY
INHIBITING SUBSTANCE

FIG.1

# FIG.2A

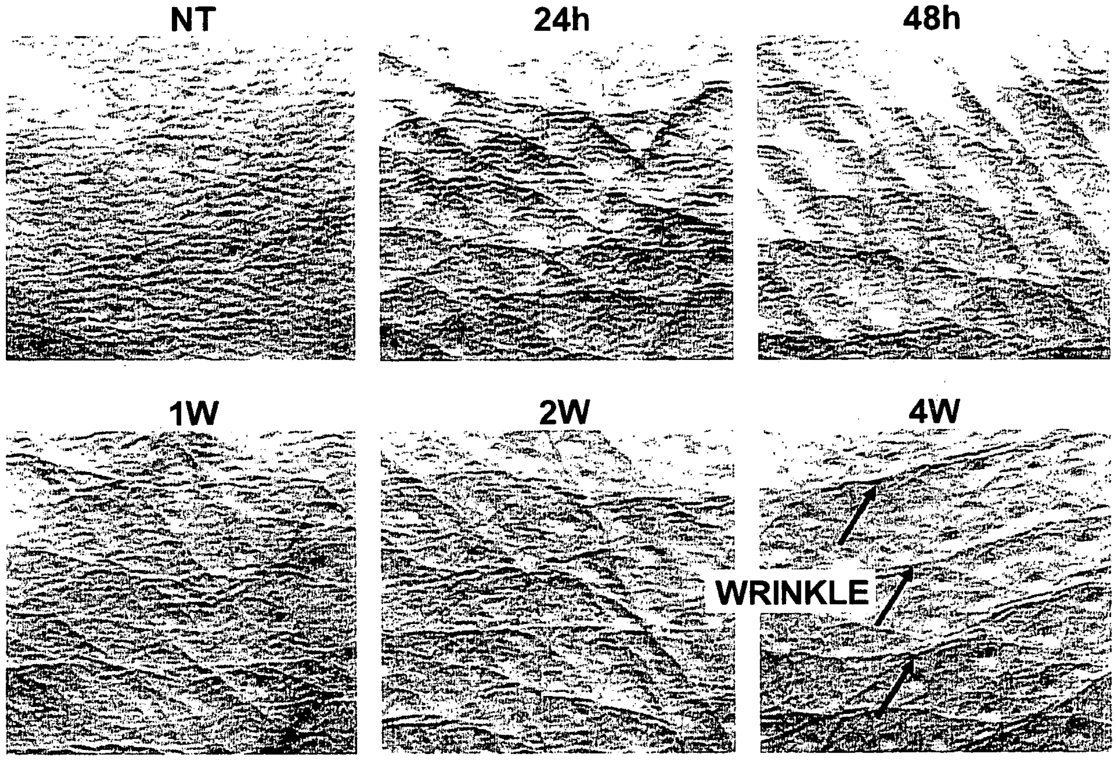


# FIG.2B

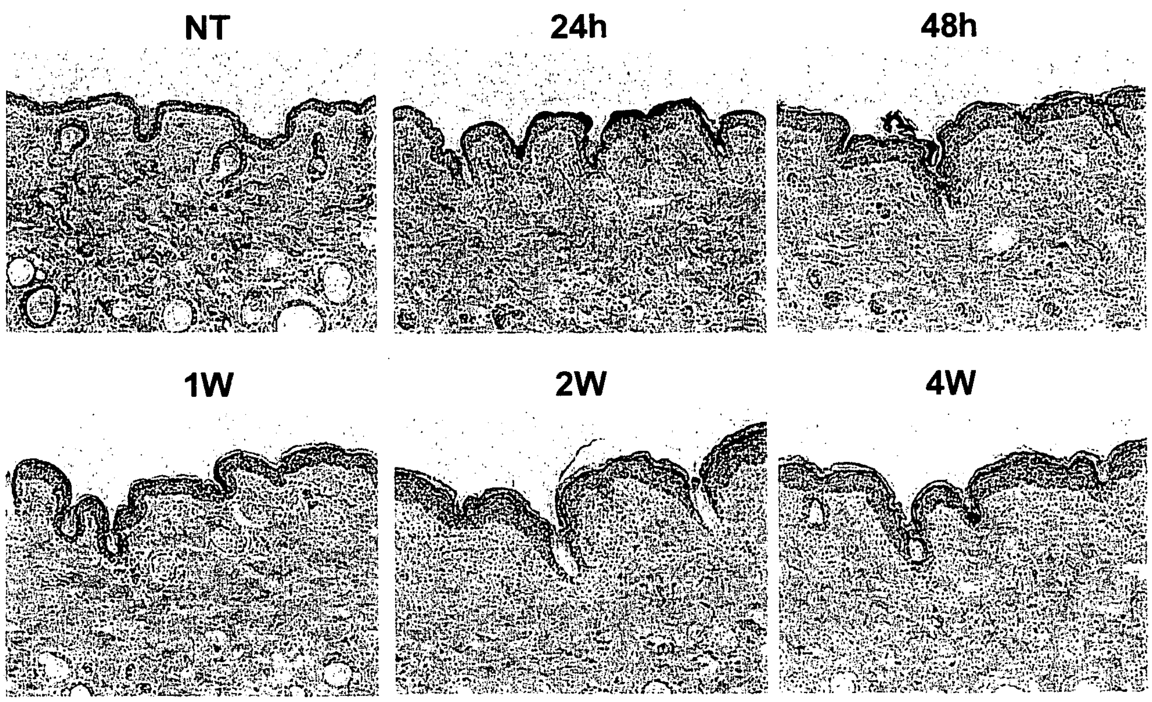

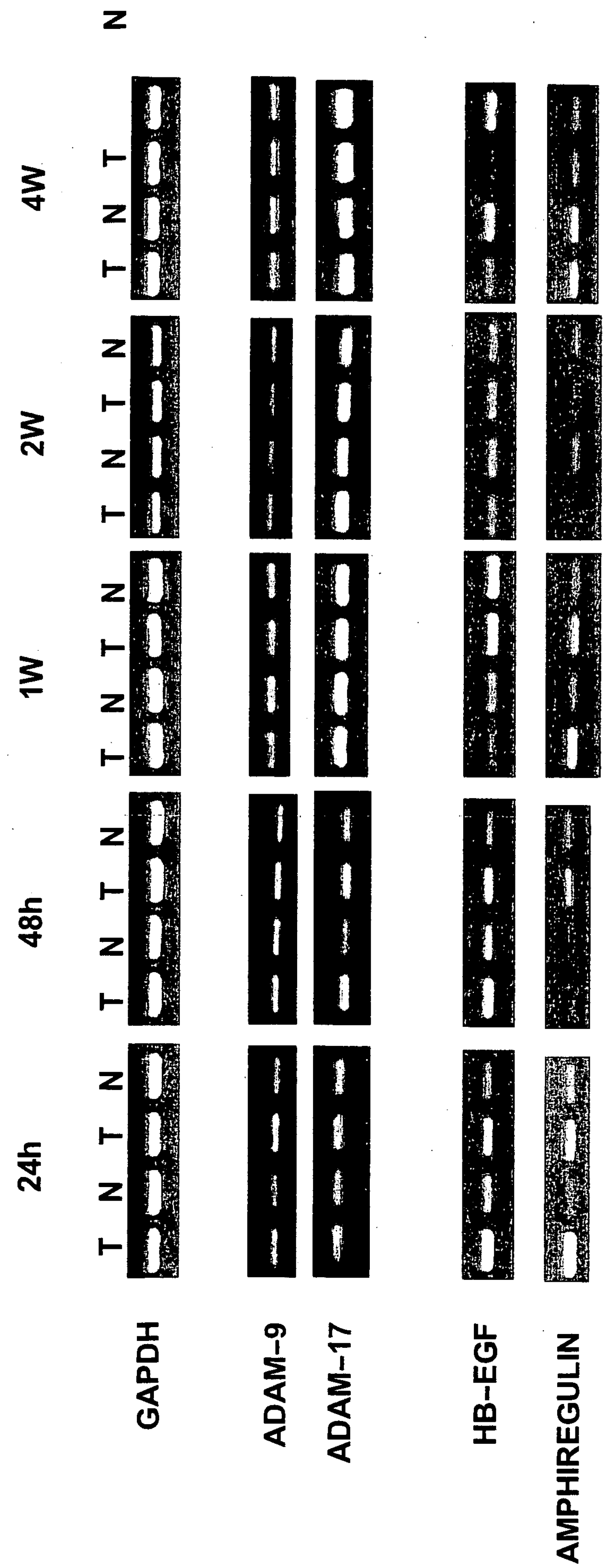


FIG.3

# FIG.4A

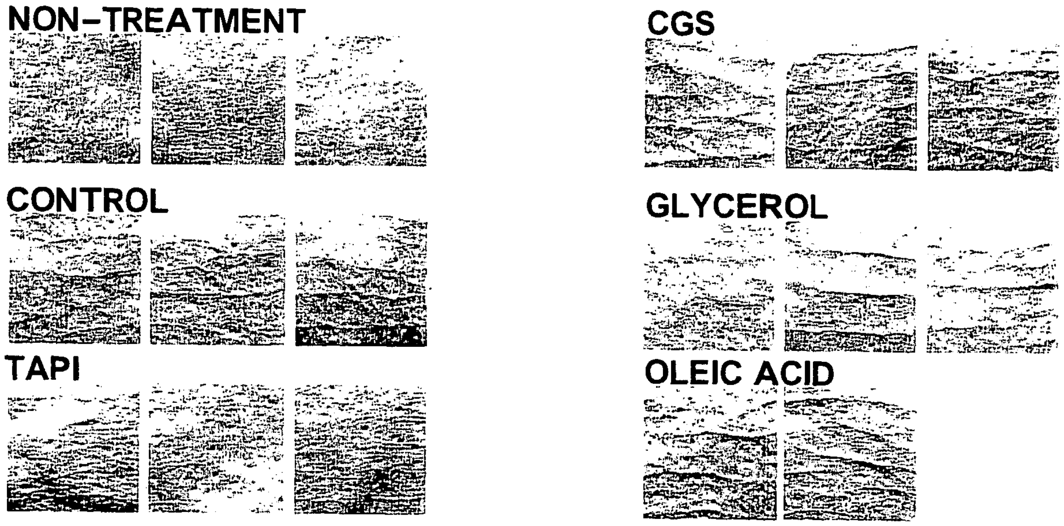
NON-TREATMENT
CGS
CONTROL
GLYCEROL
TAPI
OLEIC ACID

# FIG.4B

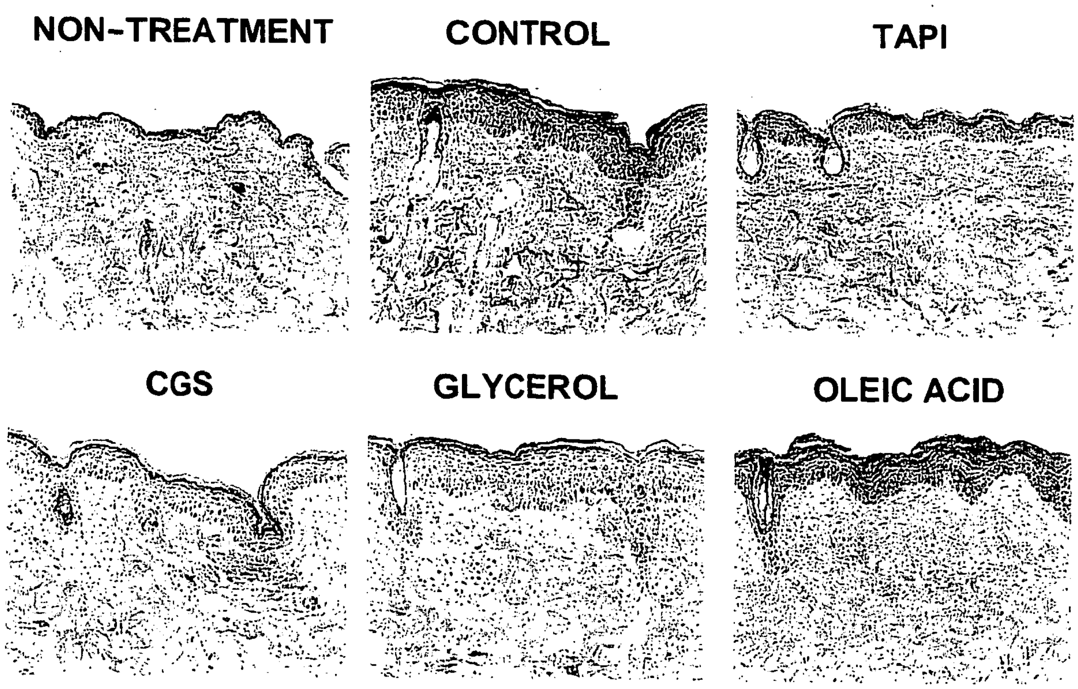
NON-TREATMENT
CONTROL
TAPI
CGS
GLYCEROL
OLEIC ACID

# FIG.5

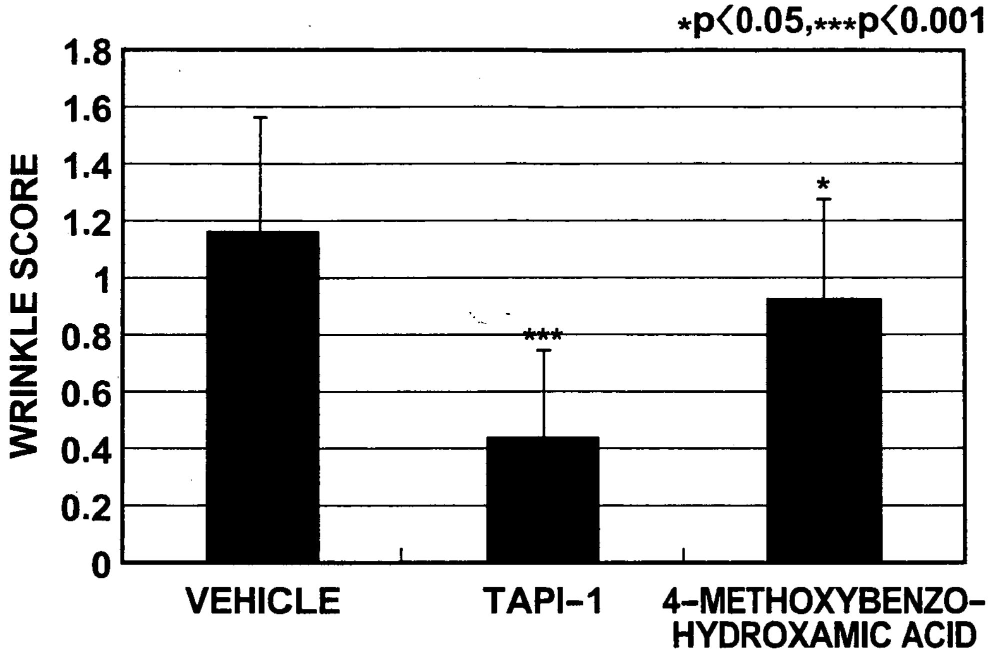
*p<0.05,***p<0.001
WRINKLE SCORE
1.8
1.6
1.4
1.2
1
0.8
0.6
0.4
0.2
0
***
*
VEHICLE
TAPI-1
4-METHOXYBENZO-HYDROXAMIC ACID

# FIG.6

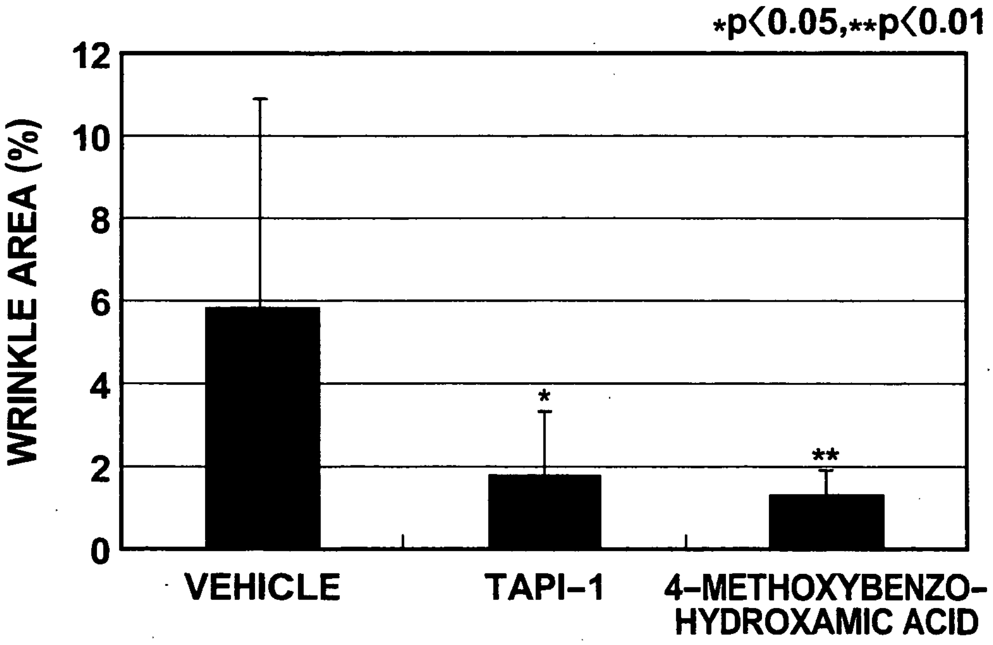
*p<0.05,**p<0.01
WRINKLE AREA (%)
12
10
8
6
4
2
0
*
**
VEHICLE
TAPI-1
4-METHOXYBENZO-HYDROXAMIC ACID

**INTERNATIONAL SEARCH REPORT**

International application No.
PCT/JP2005/019266

A. CLASSIFICATION OF SUBJECT MATTER
***A61K8/00***(2006.01), ***A61Q19/00***(2006.01), ***G01N33/50***(2006.01)

According to International Patent Classification (IPC) or to both national classification and IPC

B. FIELDS SEARCHED

Minimum documentation searched (classification system followed by classification symbols)
A61K8/00-8/99, A61Q1/00-99/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS/MEDLINE/WPIDS(STN), CAplus(STN), EMBASE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | Moriyama et al., "Azasugar-based MMP/ADAM inhibitors as antipsoriatic agents", J.Med. Chem. Vol.47, No.8, pages 1930 to 1938, (2004), full text | 1-10 |
| A | JP 2004-205246 A (Kanebo, Ltd.), 22 July, 2004 (22.07.04), Full text (Family: none) | 1-10 |
| A | WO 2003/057184 A2 (INTEGRIDERM INC), 17 July, 2003 (17.07.03), Full text & US 2003/199558 A1 & AU 2002364203 A1 & EP 1461010 A2 & KR 2004102354 A | 1-10 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 31 January, 2006 (31.01.06) | 07 February, 2006 (07.02.06) |
| Name and mailing address of the ISA/ Japanese Patent Office | Authorized officer |
| Facsimile No. | Telephone No. |

Form PCT/ISA/210 (second sheet) (April 2005)

INTERNATIONAL SEARCH REPORT

International application No.
PCT/JP2005/019266

<Subject of search>

Claims 1 to 7 relate to an agent for preventing or improving wrinkles which comprises as the active ingredient a compound that is defined by a desired property "a substance inhibiting the activity of ADAM". Although claims 1 to 7 involve any compounds having the above property, nothing but two compounds, i.e., TAPI-1 and 4-methoxybenzohydroxamic acid is disclosed in the meaning within PCT Article 5. Therefore, it is recognized that these claims are not supported by the disclosure of the description in the meaning within PCT Article 6.

Even though the common technical knowledge at the point of the application is taken into consideration, it appears that the scope of the compounds having the property "a substance inhibiting the activity of ADAM" could not be specified. Thus, claims 1 to 7 do not comply with the requirement of clearness under PCT Article 6 too.

Such being the case, the search was made on the relationship between the inhibition of the ADAM activity and wrinkles and agents for preventing or improving wrinkles which comprise the two compounds specifically cited in the description as the active ingredient.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001508809 A
- JP 2001520677 A


### Non-patent literature cited in the description


- **IMOKAWA et al.** *Fragrance Journal,* 1992, 29-42
- **JINMASAKI.** *Perfume Transactions,* 2001, vol. 25 (1), 34-38
- *J. Med. Chem.,* 1997, vol. 40, 2525-2532